(19) Europäisches Patentamt
European Patent Office
Office européen des brevets

(11) Veröffentlichungsnummer: **0 059 685**
**A1**

(12) # EUROPÄISCHE PATENTANMELDUNG

(21) Anmeldenummer: 82810084.2

(22) Anmeldetag: 22.02.82

(51) Int. Cl.³: **C 07 C 69/65**
C 07 C 67/14, C 07 C 121/75
C 07 C 120/00, A 01 N 37/10

(30) Priorität: 26.02.81 CH 1297/81
13.10.81 CH 6539/81

(43) Veröffentlichungstag der Anmeldung: 08.09.82 Patentblatt 82/36

(84) Benannte Vertragsstaaten: AT BE CH DE FR GB IT LI NL

(71) Anmelder: CIBA-GEIGY AG
Patentabteilung Postfach
CH-4002 Basel(CH)

(72) Erfinder: Ackermann, Peter, Dr.
Reichensteinerstrasse 12
CH-4153 Reinach(CH)

(72) Erfinder: Gsell, Laurenz, Dr.
Maiengasse 56
CH-4056 Basel(CH)

(72) Erfinder: Wehrli, Rudolf, Dr.
Dianastrasse 2
CH-4310 Rheinfelden(CH)

(54) **Alpha-Isopropyl- resp. alpha-Cyclopropylphenylacetate, Verfahren zu ihrer Herstellung und ihre Verwendung in der Schädlingsbekämpfung.**

(57) α-Isopropyl- resp. α-Cyclopropylphenylacetate der Formel

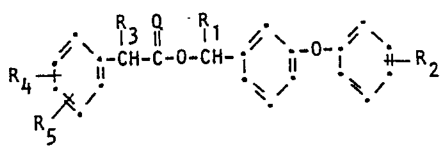

worin

$R_1$ Wasserstoff, Cyano, $-CSNH_2$, Allenyl, gegebenenfalls substituiertes $C_2$-$C_3$-Alkenyl oder $C_2$-$C_3$-Alkinyl,
$R_2$ gegebenenfalls substituiertes Alkenyl oder Alkinyl,
$R_3$ Isopropyl, Isopropenyl oder Cyclopropyl und
$R_4$ und $R_5$ je Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy oder zusammen den Methylendioxyrest bedeuten.

Ein Verfahren zur Herstellung dieser α-Isopropyl-, resp. α-Cyclopropylphenylacetate und ihre Verwendung in der Schädlingsbekämpfung werden beschrieben.

EP 0 059 685 A1

Croydon Printing Company Ltd.

0059685



CIBA-GEIGY AG
Basel (Schweiz)

5-13305/1+2/+

# α-Isopropyl- resp. α-Cyclopropylphenylacetate, Verfahren zu ihrer Herstellung und ihre Verwendung in der Schädlingsbekämpfung

Die vorliegende Erfindung betrifft α-Isopropyl- resp. α-Cyclopropylphenylacetate, Verfahren zu ihrer Herstellung und ihre Verwendung in der Schädlingsbekämpfung.

Die α-Isopropyl- resp. α-Cyclopropylphenylacetate haben die Formel

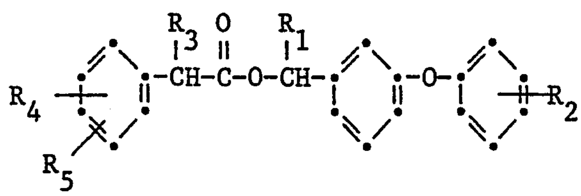

(I),

worin

$R_1$ Wasserstoff, Cyano, $-CSNH_2$, Allenyl, $C_2$-$C_3$-Alkenyl oder $C_2$-$C_3$-Alkinyl,

$R_2$ gegebenenfalls substituiertes Alkenyl oder Alkinyl,

$R_3$ Isopropyl oder Cyclopropyl und

$R_4$ und $R_5$ je Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy oder zusammen den Methylendioxyrest bedeuten.

Die für $R_2$ in Frage kommenden Alkenyl- oder Alkinylgruppen können geradkettig oder verzweigt sein und haben in der Kette bevorzugt 2 bis 6, insbesondere aber 2 bis 3 Kohlenstoffatome, Beispiele von bevorzugten Substituenten an diesen Gruppen sind: Cyano, Halogen, wie Fluor, Chlor, Brom, Jod, $C_1$-$C_6$-Alkyl, $C_1$-$C_6$-Hydroxyalkyl, $C_1$-$C_6$-Halogenalkyl, $C_1$-$C_6$-Alkoxy, $C_1$-$C_6$-Alkylthio, Dimethylamino oder Phenyl. Besonders bevorzugte Gruppen bei $R_2$ sind Vinyl, 1-Propenyl, 1,2-Dibromvinyl, Aethinyl, 2-Jodäthinyl oder 1-Propinyl.

Unter Halogen bei $R_4$ und $R_5$ ist Fluor, Chlor, Brom oder Jod, insbesondere aber Chlor, zu verstehen. Die Alkyl-, Halogenalkyl-, Alkoxy- und Halogenalkoxygruppen bei $R_4$ und $R_5$ können verzweigt oder geradkettig sein. Bevorzugte Beispiele solcher Gruppen sind: Methyl, Trifluormethyl, Methoxy, Difluormethoxy, Aethyl, Aethoxy oder Isopropyl und tert.Butyl.

Bevorzugt sind Verbindungen der Formel I, worin $R_1$ Wasserstoff, Cyano, $-CSNH_2$, $C_2$-$C_3$-Alkenyl oder $C_2$-$C_3$-Alkinyl, $R_2$ Alkenyl oder Alkinyl, $R_3$ Isopropyl oder Cyclopropyl und $R_4$ und $R_5$ je Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy oder zusammen den Methylendioxyrest bedeuten.

Ebenfalls bevorzugt sind Verbindungen der Formel I, worin $R_1$ Wasserstoff, Cyano, Allenyl oder Aethinyl, $R_2$ Vinyl, 1-Propenyl, Aethinyl, 2-Jodäthinyl oder 1-Propinyl, $R_3$ Isopropyl oder Cyclopropyl, $R_4$ Halogen, $C_1$-$C_4$-Alkyl, Trifluormethyl, $C_1$-$C_4$-Alkoxy oder Difluormethoxy und $R_5$ Wasserstoff oder Halogen bedeuten.

Besonders bevorzugt sind Verbindungen der Formel I, worin $R_1$ Wasserstoff oder Cyano, $R_2$ Vinyl, Aethinyl oder 1-Propinyl, $R_3$ Isopropyl oder Cyclopropyl, $R_4$ Fluor, Chlor, Brom, Methyl, Trifluormethyl, Methoxy oder Difluormethoxy, und $R_5$ Wasserstoff bedeuten.

Insbesondere bevorzugt sind aber Verbindungen der Formel I, worin $R_1$ Cyano, $R_2$ p-Vinyl, p-1-Propenyl, p-Aethinyl oder p-1-Propinyl, $R_3$ Isopropyl oder Cyclopropyl, $R_4$ p-Chlor und $R_5$ Wasserstoff bedeuten.

Die Verbindungen der Formel I können nach an sich bekannten Methoden hergestellt werden:

1) 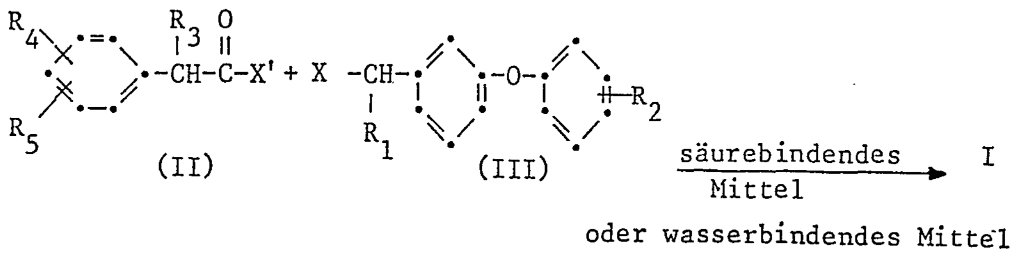

(II) (III) I

2) 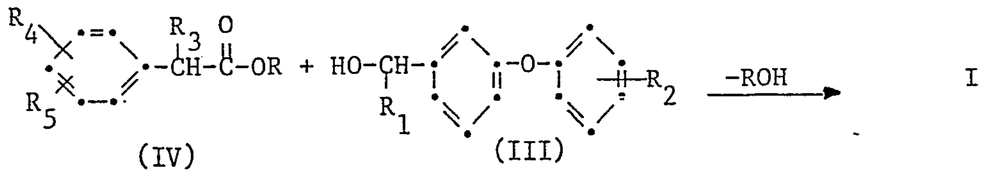

(IV) (III) I

In den Formeln II bis IV haben $R_1$ bis $R_5$ die für die Formel I angegebene Bedeutung. In der Formeln II und III stehen die Symbole X und X' je für eine Hydroxylgruppe oder eines der Symbole X' oder X für eine Hydroxylgruppe und das andere für ein Halogenatom, insbesondere für Chlor oder Brom und in der Formel IV steht R für $C_1$-$C_4$-Alkyl, insbesondere für Methyl oder Aethyl.

Als säurebindendes Mittel für das Verfahren 1 kommen insbesondere tertiäre Amine, wie Trialkylamin und Pyridin, ferner Hydroxide, Oxide, Carbonate und Bicarbonate von Alkali- und Erdalkalimetallen sowie Alkalimetallalkoholate wie z.B. Kalium-t.butylat und Natriummethylat in Betracht. Als wasserbindendes Mittel für das Verfahren 1 kann z.B. Dicyclohexylcarbodiimid verwendet werden. Die Verfahren 1 und 2 werden bei einer Reaktionstemperatur zwischen -10 und 120°C, meist zwischen 20 und 80°C, bei normalem oder erhöhtem Druck und vorzugsweise in einem inerten Lösungs oder Verdünnungsmittel durchgeführt. Als Lösungs- oder Verdünnungsmittel eignen sich z.B. Aether und ätherartige Verbindungen wie Diäthyläther, Dioxan, Dimethoxyäthan und Tetrahydrofuran; Amide wie N,N-dialkylierte Carbonsäureamide; aliphatische, aromatische sowie halogenierte Kohlenwasserstoffe, insbesondere Benzol, Toluol, Xylol, Chloroform und Chlorbenzol; Nitrile wie

Acetonitril; Dimethylsulfoxid und Ketone wie Aceton und Methyläthylketon.

Die Ausgangsstoffe der Formeln II bis V sind bekannt und können analog bekannten Methoden hergestellt werden.

Die Verbindungen der Formel I liegen als Gemisch von verschiedenen optisch aktiven Isomeren vor, wenn bei der Herstellung nicht einheitlich optisch aktive Ausgangsmaterialien verwendet werden. Die verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen Schädlingen an Tieren und Pflanzen.

So eignen sich die Verbindungen der Formel I zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, und von Milben und Zecken der Ordnung Akarina.

Vor allem eignen sich die Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z.B. Spodoptera littoralis und Heliothis virescens), Gemüsekulturen (z.B. Leptinotarsa decemlineata und Myzus persicae), Reiskulturen (z.B. Chilo suppressalis und Laodelphax striatellus)und Obstkulturen (z.B. Laspeyresia pomonella und Adoxophyes).

Die Wirkstoffe der Formel I zeigen auch eine sehr günstige Wirkung gegen Fliegen, wie z.B. Musca domestica und Mückenlarven.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. organische Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden die Verbindungen der Formel I auch mit Substanzen kombiniert, welche einen synergistischen oder verstärkenden Effekt auf Pyrethroide ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphortrithionate, 1,2-Methylendioxy-4-(2-octylsulfinyl)-propyl)-benzol.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/ oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}-C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im(aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben:

"Mc Cutcheon's Detergents and Emulsifiers Annual" MC Publishing Corp., Ringwood, New Jersey, 1979.

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I

(% = Gewichtsprozent)

| 1. Emulsions-Konzentrate | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | - | - |
| Tributylphenol-polyäthylenglykoläther (30 Mol AeO) | - | 12% | 4,2% |
| Cyclohexanon | - | 15% | 20% |
| Xylolgemisch | 70% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80% | 10% | 5% | 95% |
| Aethylenglykol-monomethyl-äther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

| 3. Granulate | a) | b) |
|---|---|---|
| Wirkstoff | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I
(% = Gewichtsprozent)

| 5. Spritzpulver | a) | b) |
|---|---|---|
| Wirkstoff | 20% | 60% |
| Na-Ligninsulfonat | 5% | 5% |
| Na-Laurylsulfat | 3% | - |
| Na-Diisobutylnaphthalinsulfonat | - | 6% |
| Octylphenolpolyäthylenglykoläther (7-8 Mol AeO) | - | 2% |
| Hochdisperse Kieselsäure | 5% | 27% |
| Kaolin | 67% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

8. Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulat

| | |
|---|---|
| Wirkstoff | 3% |
| Polyäthylenglykol (M G 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1: Herstellung von 2-(4-Chlorphenyl)-3-methyl-buttersäure-α-cyano-3-(4-äthinylphenoxy)-benzylester.

Zu einer Lösung von 2,3 g der Verbindung der Formel

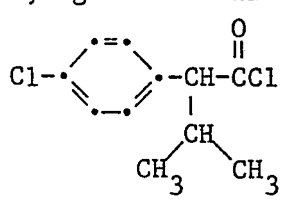

in 20 ml Toluol tropft man bei 0°C nacheinander 1 ml Pyridin und 5,4 g der Verbindung der Formel

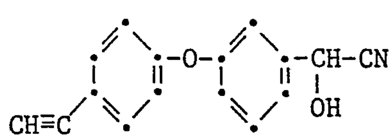

gelöst in 20 ml Toluol. Nach 12stündigem Rühren bei 20°C wird das Reaktionsgemisch auf 2n Salzsäure gegossen, je einmal mit 10%iger Kaliumkarbonat-, gesättigter Natriumbicarbonat- und gesättigter Kochsalzlösung extrahiert. Die organische Phase wird mit Magnesiumsulfat getrocknet, filtriert und eingeengt. Nach der Chromatographie des Rohproduktes an Kieselgel mit Toluol als Eluiermittel erhält man die Verbindung der Formel

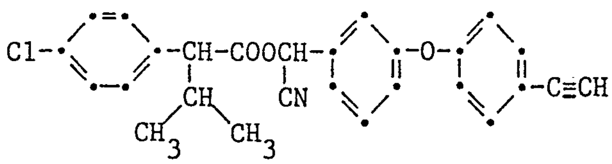

mit einer Refraktion von $n_D^{20°} = 1,5811$.

Auf analoge Weise werden auch folgende Verbindungen hergestellt:

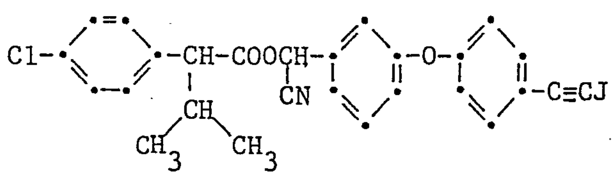

$n_D^{30°} = 1,5969$

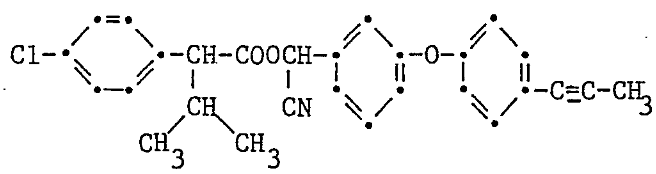

$n_D^{30°} = 1,5767$

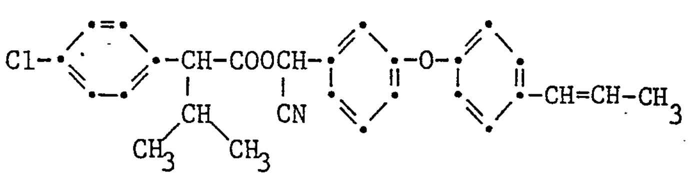

$n_D^{20°} = 1,5752$

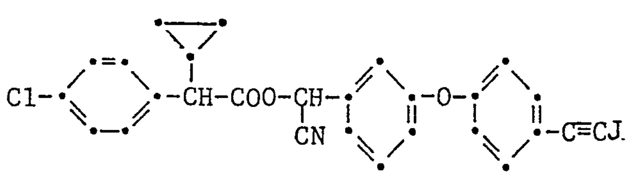

$n_D^{30°} = 1,6116$

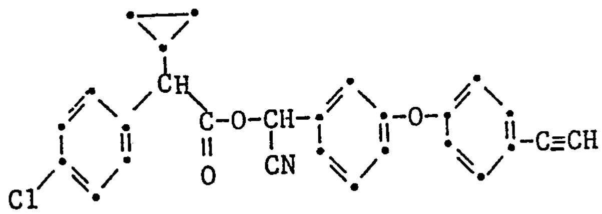

$n_D^{25°} = 1,5901$

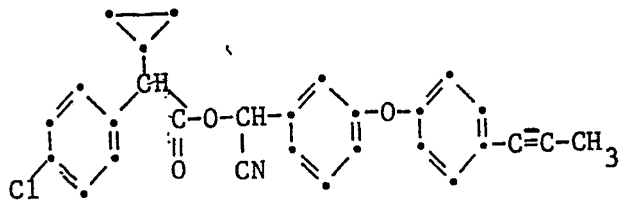

$n_D^{30°} = 1,5858$

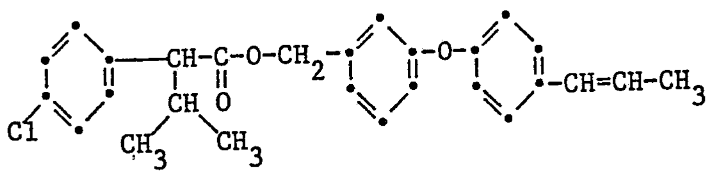

$n_D^{20°} = 1,5804$

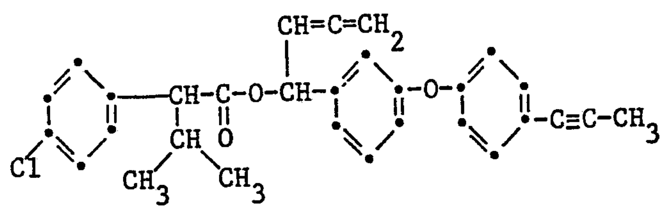

$n_D^{30°} = 1,5912$

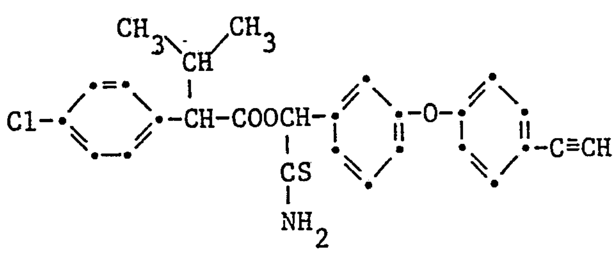

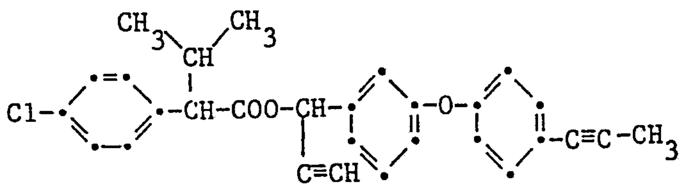

$n_D^{20°} = 1,5813$

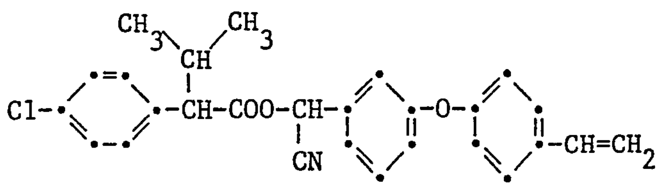

$n_D^{30°} = 1,5747$

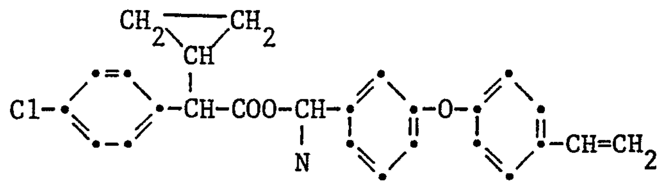

$n_D^{30°} = 1,5868$

Beispiel 2: Insektizide Frassgift-Wirkung: Laspeyresia pomonella

10 Diätwürfel werden mit einer Versuchslösung, enthaltend 10, 50, 100, 200 oder 400 ppm der zu prüfenden Verbindung, besprüht.

Nach dem Antrocknen des Belages werden die Diätwürfel mit je 2 Larven der Spezies Laspeyresia pomonella ($L_1$-Stadium) besetzt. Die Auswertung der erzielten Abtötungsrate erfolgt nach 48 Stunden. Der Versuch wurde bei 24°C und 60% relativer Luftfeuchtigkeit durchgeführt.

Die Verbindung gemäss dem Herstellungsbeispiel 1 haben die in der folgenden Tabelle angegebene Wirkung gegenüber den Larven von Laspeyresia pomonella.

Biologische Versuchsergebnisse

In der folgenden Tabelle sind Versuchsergebnisse auf der Basis der vorstehenden Beispiele aufgeführt, und zwar mit folgendem Bewertungs-Index in Bezug auf die prozentuale Abtötung der Schädlinge:

A: 70-100% Abtötung bei 10 ppm Wirkstoffkonzentration
B: 70-100% Abtötung bei 50 ppm Wirkstoffkonzentration
C: 70-100% Abtötung bei 100 ppm Wirkstoffkonzentration
D: 70-100% Abtötung bei 200 ppm Wirkstoffkonzentration
E: 70-100% Abtötung bei 400 ppm Wirkstoffkonzentration

| Verbindung 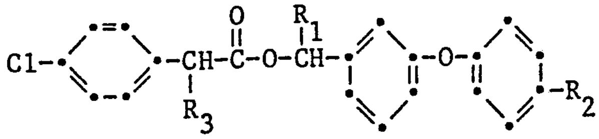 | | | Wirksamkeit gegen $L_1$ Larven von Laspeyresia pomonella |
|---|---|---|---|
| $R_1$ | $R_2$ | $R_3$ | |
| -CN | -C≡CH | $-C_3H_{7(i)}$ | A |
| -CN | -C≡CJ | $-C_3H_{7(i)}$ | B |
| -CN | $-C≡C-CH_3$ | $-C_3H_{7(i)}$ | B |
| -CN | $-CH=CH-CH_3$ | $-C_3H_{7(i)}$ | B |
| -CN | -C≡CJ | Cyclopropyl | B |
| -CN | -C≡CH | Cyclopropyl | B |
| -CN | $-C≡C-CH_3$ | Cyclopropyl | B |
| H | $-CH=CH-CH_3$ | $-C_3H_{7(i)}$ | E |
| $-CH=C=CH_2$ | $-C≡C-CH_3$ | $-C_3H_{7(i)}$ | D |
| -C≡CH | $-C≡C-CH_3$ | $-C_3H_{7(i)}$ | D |
| -CN | $-CH=CH_2$ | $-C_3H_{7(i)}$ | A |
| -CN | $-CH=CH_2$ | Cyclopropyl | B |

Patentansprüche

1. α-Isopropyl- resp. α-Cyclopropylphenylacetate der Formel

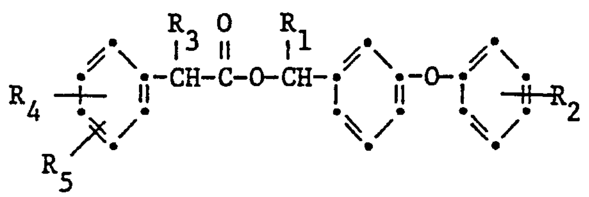

(I),

worin

$R_1$ Wasserstoff, Cyano, $-CSNH_2$, Allenyl, $C_2$-$C_3$-Alkenyl oder $C_2$-$C_3$-Alkinyl,

$R_2$ gegebenenfalls substituiertes Alkenyl oder Alkinyl,

$R_3$ Isopropyl oder Cyclopropyl und

$R_4$ und $R_5$ je Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy oder zusammen den Methylendioxyrest bedeuten.

2. Eine Verbindung gemäss Anspruch 1, worin

$R_1$ Wasserstoff, Cyano, $-CSNH_2$, $C_2$-$C_3$-Alkenyl oder $C_2$-$C_3$-Alkinyl,

$R_2$ Alkenyl oder Alkinyl,

$R_3$ Isopropyl oder Cyclopropyl und

$R_4$ und $R_5$ je Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy oder zusammen den Methylendioxyrest bedeuten.

3. Eine Verbindung gemäss Anspruch 1, worin

$R_1$ Wasserstoff, Cyano, Allenyl oder Aethinyl,

$R_2$ Vinyl, Aethinyl, 2-Jodäthinyl oder n-Propinyl,

$R_3$ Isopropyl oder Cyclopropyl,

$R_4$ Halogen, $C_1$-$C_4$-Alkyl, Trifluormethyl, $C_1$-$C_4$-Alkoxy oder Difluormethoxy und

$R_5$ Wasserstoff oder Halogen bedeuten.

4. Eine Verbindung gemäss Anspruch 3, worin
$R_1$ Wasserstoff oder Cyano,
$R_2$ Vinyl, 1-Propenyl, Aethinyl oder 1-Propinyl,
$R_3$ Isopropyl oder Cyclopropyl,
$R_4$ Fluor, Chlor, Brom, Methyl, Trifluormethyl, Methoxy oder Difluormethoxy und
$R_5$ Wasserstoff bedeuten.

5. Eine Verbindung gemäss Anspruch 4, worin
$R_1$ Cyano,
$R_2$ p-Vinyl, p-Aethinyl oder p-1-Propinyl,
$R_3$ Isopropyl oder Cyclopropyl,
$R_4$ p-Chlor und
$R_5$ Wasserstoff bedeuten.

6. Die Verbindung gemäss Anspruch 5 der Formel

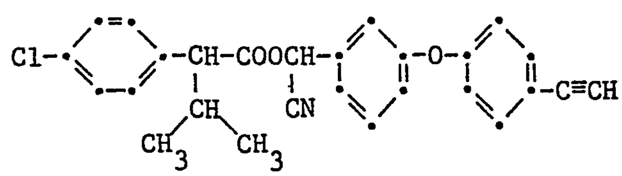
.

7. Die Verbindung gemäss Anspruch 5 der Formel

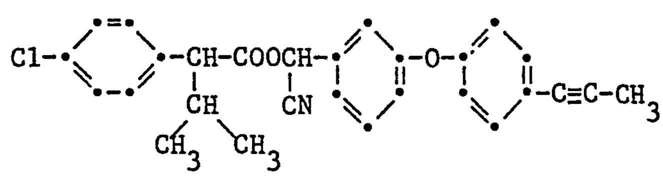
.

8. Die Verbindung gemäss Anspruch 5 der Formel

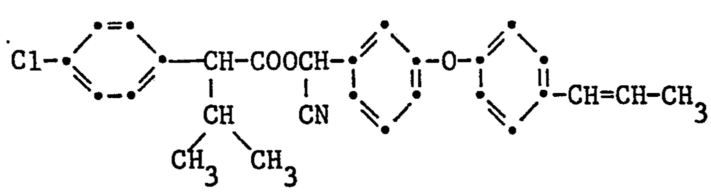
.

9. Die Verbindung gemäss Anspruch 5 der Formel

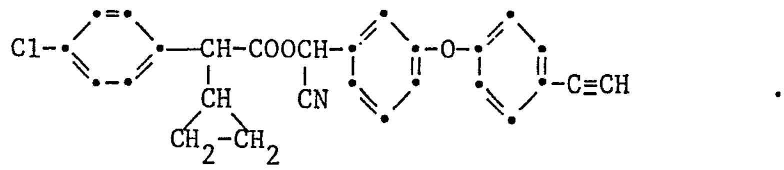

10. Die Verbindung gemäss Anspruch 5 der Formel

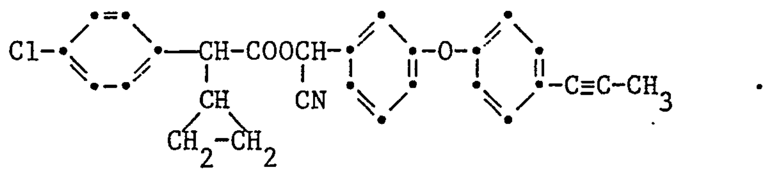

11. Die Verbindung gemäss Anspruch 4 der Formel

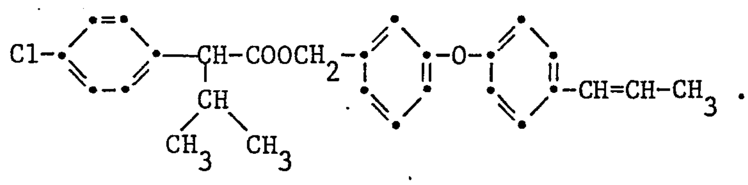

12. Die Verbindung gemäss Anspruch 3 der Formel

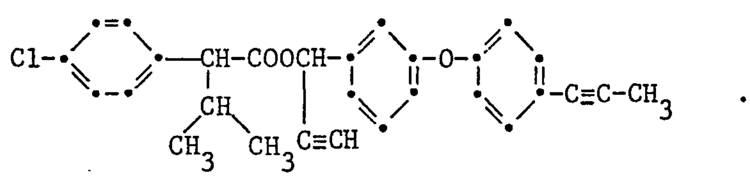

13. Die Verbindung gemäss Anspruch 3 der Formel

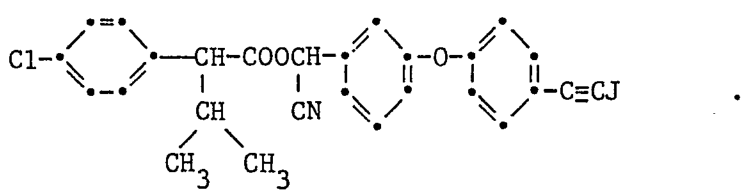

14. Die Verbindung gemäss Anspruch 3 der Formel

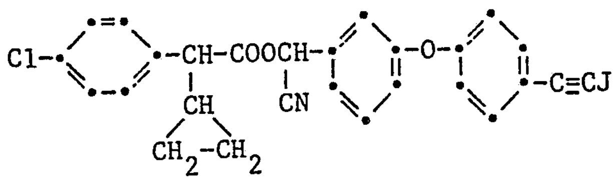

15. Die Verbindung gemäss Anspruch 3 der Formel

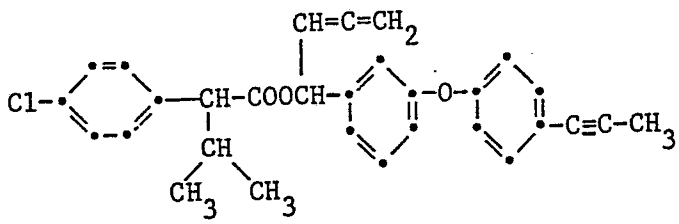

16. Die Verbindung gemäss Anspruch 5 der Formel

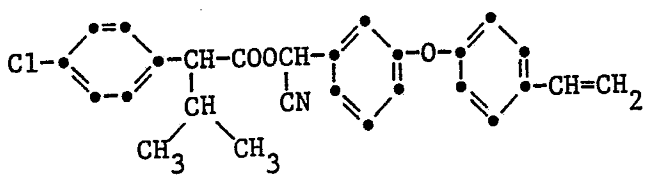

17. Ein Verfahren zur Herstellung von Verbindungen gemäss Anspruch 1, dadurch gekennzeichnet, dass man in Gegenwart eines säuresbindenden Mittels eine Verbindung der Formel

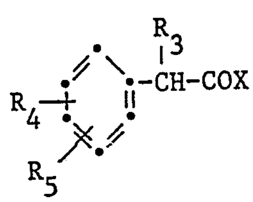

mit einer Verbindung der Formel

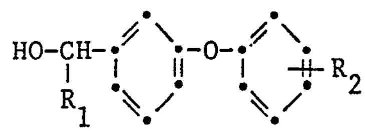

umsetzt, worin $R_1$ bis $R_5$ die im Anspruch 1 angegebene Bedeutung haben und X für ein Halogenatom steht.

18. Ein Schädlingsbekämpfungsmittel, welches als aktive Komponente eine Verbindung gemäss Anspruch 1 enthält.

19. Die Verwendung einer Verbindung gemäss Anspruch 1 zur Bekämpfung von Schädlingen an Tieren und Pflanzen.

20. Die Verwendung gemäss Anspruch 19 zur Bekämpfung von Insekten und Vertretern der Ordnung Akarina.

Europäisches Patentamt

# EUROPÄISCHER RECHERCHENBERICHT

0059685

Nummer der Anmeldung

EP 82 81 0084

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| A | EP-A-0 006 630 (CIBA-GEIGY)<br>---<br> | | C 07 C 69/65<br>C 07 C 67/14<br>C 07 C 121/75<br>C 07 C 120/00<br>A 01 N 37/10 |
| A | EP-A-0 007 421 (CIBA-GEIGY)<br>----- | | |
| | | | RECHERCHIERTE SACHGEBIETE (Int. Cl. 3) |
| | | | C 07 C 69/00<br>C 07 C 67/00<br>C 07 C 121/00<br>C 07 C 120/00 |

Der vorliegende Recherchenbericht wurde für alle Patentansprüche erstellt.

| Recherchenort | Abschlußdatum der Recherche | Prüfer |
|---|---|---|
| DEN HAAG | 01-06-1982 | KINZINGER J.M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82